# EUROPEAN PATENT APPLICATION

(11) **EP 3 834 718 A1**
(43) Date of publication of application: **16.06.2021**
(21) Application number: 20212550.6
(22) Date of filing: 08.12.2020
(51) Int. Cl.: A61B 5/06, A61F 2/95, A61M 25/00, A61F 2/24

(54) **DETERMINING RELEASE OF IMPLANT FROM SHEATH BASED ON MEASURING IMPEDANCE**

(30) Priority: 09.12.2019 US 201916706997
(71) Applicant: Biosense Webster (Israel) Ltd., 2066717 Yokneam (IL)
(72) Inventor: GOVARI, Assaf, 2066717 Yokneam (IL)
(74) Representative: Small, Gary James

(57) **Abstract**

An apparatus includes an electrical conductor, a patch electrode and readout circuitry. The electrical conductor is configured to be connected to an implant fitted at a distal end of a shaft for insertion via a sheath into a liquid-filled lumen of an organ of a patient, the patch electrode that is configured to be attached to skin of the patient. The readout circuitry is configured to be connected to the electrical conductor and to the patch electrode, to measure, via the electrical conductor, an electrical impedance between the implant and the patch electrode, and to detect the implant exiting the sheath into the lumen by detecting a drop in the electrical impedance that is larger than a predefined threshold.

## Description

### FIELD OF THE INVENTION

The present invention relates generally to monitoring invasive medical procedures, and particularly to monitoring deployment of implants.

### BACKGROUND OF THE INVENTION

Various techniques were proposed in the patent literature to assist invasive procedures such as delivery and placement of an implant using a probe. For example, U.S. Patent Application Publication 2003/0187340 describes a first electrode positioned within an artery proximate an implanted intravascular stent. A second electrode is positioned at a separate location relative the position of the first electrode. Electrical energy is then delivered between the first and the second electrodes to produce an electrical field adjacent the implanted intravascular stent. When an intravascular stent is implanted in a coronary artery, the delivery of the electrical energy is coordinated to cardiac cycles detected in sensed cardiac signals, where the delivery of the electrical energy between the first electrode and the second electrode occurs during a predetermined portion of the cardiac cycle.

As another example, U.S. Patent Application Publication 2015/0038833 describes methods and systems for determining information about a vascular bodily lumen. An exemplary method includes generating an electrical signal, delivering the electrical signal to a plurality of excitation elements in the vicinity of the vascular bodily lumen, measuring a responsive electrical signal from a plurality of sensing elements in response to the delivered electrical signal, and determining a lumen dimension. Specific embodiments include using spatial diversity of the excitation elements. Diagnostic devices incorporating the method are also disclosed, including guidewires, catheters and implants. The methods and systems described herein are advantageous as they do not include injecting a second fluid for the measurements.

### SUMMARY OF THE DISCLOSURE

An embodiment of the present invention provides an apparatus including an electrical conductor, a patch electrode and readout circuitry. The electrical conductor is configured to be connected to an implant fitted at a distal end of a shaft for insertion via a sheath into a liquid-filled lumen of an organ of a patient. the patch electrode that is configured to be attached to skin of the patient. The readout circuitry is configured to be connected to the electrical conductor and to the patch electrode, to measure, via the electrical conductor, an electrical impedance between the implant and the patch electrode, and to detect the implant exiting the sheath into the lumen by detecting a drop in the electrical impedance that is larger than a predefined threshold.

In some embodiments, the apparatus further includes a magnetic sensor fitted at the distal end of the shaft, adjacently to the implant, the magnetic sensor configured to transmit position signals indicative of a position of the implant when the implant exits the sheath into the lumen.

In an embodiment, the liquid is blood. In another embodiment, the organ is a heart.

In some embodiments, the implant is an artificial heart-valve. In other embodiments, the implant is a stent.

There is additionally provided, in accordance with an embodiment of the present invention, a method including connecting an electrical conductor to an implant fitted at a distal end of a shaft for insertion via a sheath into a liquid-filled lumen of an organ of a patient. A patch electrode is attached to skin of the patient. An electrical impedance is measured, via the electrical conductor, between the implant and the patch electrode, and the implant exiting the sheath into the lumen is detected by detecting a drop in the electrical impedance that is larger than a predefined threshold.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based position-tracking system, in accordance with an embodiment of the present invention;
Figs. 2A and 2B are schematic side views of a stent positioned inside and outside of a distal end of a sheath, respectively, in accordance with an embodiment of the present invention;
Fig. 3 is a graph that schematically shows impedances measured between the stent of Figs. 2A and 2B and a patch electrode, respectively, in accordance with an embodiment of the present invention; and
Fig. 4 is a flow chart that schematically describes a method to determine the status of the stent shown in Figs. 2A and 2B, in accordance with an embodiment of the present invention.

### DETAILED DESCRIPTION OF EMBODIMENTS

### OVERVIEW

Placement of medical implants, such as cardiac stents and valves, typically requires monitoring the deployment status of the implants, such as monitoring whether the implant is collapsed inside a distal end of a sheath of a delivery probe (e.g., a catheter) or released from the sheath. In the latter case, for example, the implant may already be at least partially in an expanded state and ready for placement. Present systems for tracking an implant, such as a stent, when it is positioned in a blood vessel, typically use X-ray fluoroscopy. However, for the health of the patient and operating personnel, it is preferable not to use a stent-tracking system that uses ionizing radiation.

In an invasive procedure involving placement of an implant, such as in a valve replacement or in a stenting procedure, a sheath is inserted into the blood vessel where the implant is to be located. Once the sheath is in place, the sheath's distal end is located using, for example, the CARTO™ system, produced by Biosense-Webster Inc. (Irvine, California), and the implant is pushed through the sheath by the probe (e.g., catheter) which is to deliver the implant.

Embodiments of the present invention that are described hereinafter provide a technique to monitor the status of the implant on a verge of exiting the sheath in order to determine its subsequent exit. In some embodiments, an electrical conductor (typically a wire) is attached to the implant (e.g., stent), and this electrical conductor is used to measure the impedance between the implant and a patch electrode attached to the patient's skin. Once the stent is deployed, the wire is cut at the stent using standard tooling (e.g., a clamp).

The impedance between the implant and the patch electrode is initially measured while the implant is in a collapsed configuration inside the sheath. As long as the implant is at least partially in the sheath and in a collapsed state, the impedance is expected to indicate that the implant surface has only minimal contact with blood, and is mostly in contact with the surrounding electrically insulating sheath. As the implant exits the sheath and becomes fully immersed in blood, the impedance is expected to reduce by an amount above a predefined threshold, since the implant is now in the blood pool (and possibly also in a partially expanded configuration that allows more contact area with the electrically conducting blood). This reduction (e.g., drop) in impedance can therefore be used as an indication that the implant is positioned distally just beyond (i.e., outside) the distal end of the sheath.

The total impedance measured between the patch electrode and the stent includes a local changing impedance between the stent and blood pool. As the implant exits the sheath, a reduction (e.g., a step-wise drop) in impedance, of several tens of ohms is expected. Compared with a baseline impedance to the patch of several hundreds of ohms, the change is measured as few tens of percent of that baseline impedance, and such magnitude of change is expected to be a reliable indicator of the implant exiting the sheath.

The disclosed technique may reduce exposure to excessive amounts of X-ray radiation to the patient and medical staff deploying an implant in a lumen of the patient.

### SYSTEM DESCRIPTION

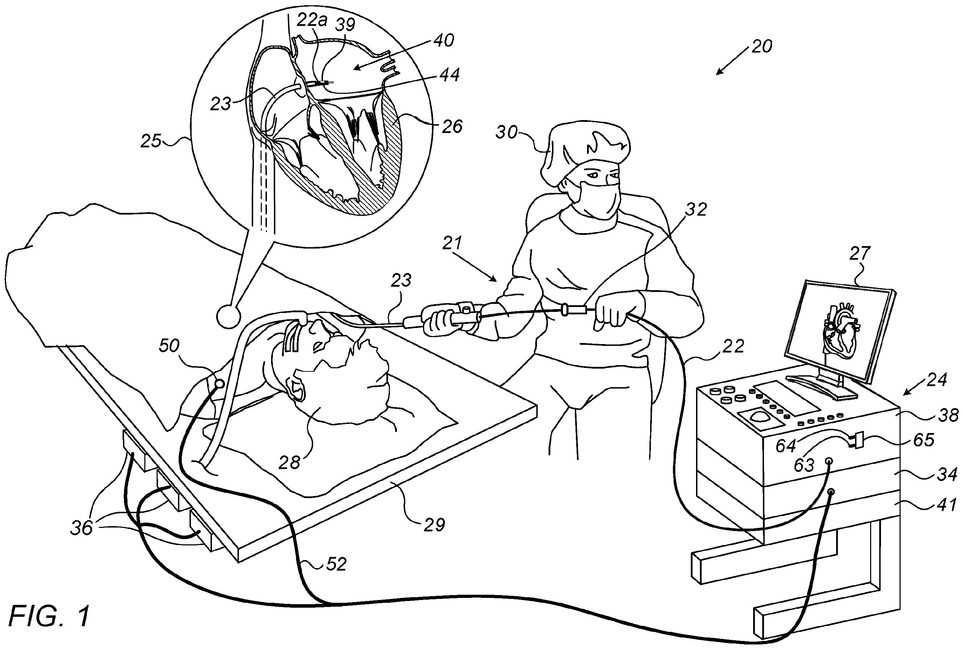

Fig. 1 is a schematic, pictorial illustration of a catheter-based position-tracking system 20, in accordance with an embodiment of the present invention. System 20 comprises a catheter 21, wherein, as seen in inset 25, a distal end 22a of shaft 22 of catheter 21 is inserted through a sheath 23 into a heart 26 of a patient 28 lying on a table 29. As further shown in inset 25, distal end 22a comprises a magnetic sensor 39, fitted at distal end 22a just proximally to a stent 40.

The proximal end of catheter 21 is connected to a control console 24. In the embodiment described herein, catheter 21 is used for placement of stent 40 in a blood-filled vessel of heart 26.

During navigation of distal end 22a in heart 26, console 24 receives signals from magnetic sensor 39 in response to magnetic fields from external field generators 36, for example, for the purpose of measuring the position of stent 40 in the heart and, optionally, presenting the tracked position on a display 27. Magnetic field generators 36 are placed at known positions external to patient 28, e.g., below patient table 29. Console 24 also comprises a driver circuit 34, configured to drive magnetic field generators 36.

In an embodiment, position signals received from position sensor 39 are indicative of the position of stent 40 in the coordinate system of position tracking and catheter-based position-tracking system 20. The method of position sensing using external magnetic fields is implemented in various medical applications, for example, in the CARTO™ system, produced by Biosense-Webster Inc. (Irvine, California), and is described in detail in U.S. Patents 5,391,199 and 6,332,089, and in PCT Patent Publication WO 96/05768, whose disclosures are all incorporated herein by reference.

Physician 30 navigates the distal end of shaft 22 to a target location in heart 26 by manipulating shaft 22 using a manipulator 32 near the proximal end of the catheter and/or deflection from the sheath 23. During the insertion of shaft 22, stent 40 is maintained in a collapsed configuration by sheath 23. By containing stent 40 in a collapsed configuration, sheath 23 also serves to minimize vascular trauma along the way to target location. A wire 44 (or other suitable electrical conductor) in shaft 22 electrically connects the implant (e.g., stent) to a first pole 63 of a readout circuitry 65 in console 24. A patch electrode 50 is attached to the patient's skin and wired to a second pole 64 of readout circuitry 65 with a wire 52. Readout circuitry 65 is configured to indicate a reduction (e.g., a step-wise drop) in an impedance measured between first pole 63 and second pole 64 of the readout circuitry, so as to indicate a status of stent 40, as described below.

Control console 24 comprises a processor 41, typically a general-purpose computer, with suitable front end and interface circuits 38 for receiving signals from catheter 21, as well as for applying treatment via catheter 21 in heart 26 and for controlling the other components of system 20. Processor 41 typically comprises a general-purpose computer with software programmed to carry out the functions described herein. The software may be downloaded to the computer in electronic form, over a network, for example, or it may, alternatively or additionally, be provided and/or stored on non-transitory tangible media, such as magnetic, optical, or electronic memory.

The example configuration shown in Fig. 1 is chosen purely for the sake of conceptual clarity. The disclosed techniques may similarly be applied using other system components and settings. For example, system 20 may comprise other components and perform non-cardiac stent implants.

### DETERMINING RELEASE OF STENT FROM SHEATH BASED ON MEASURING IMPEDANCE

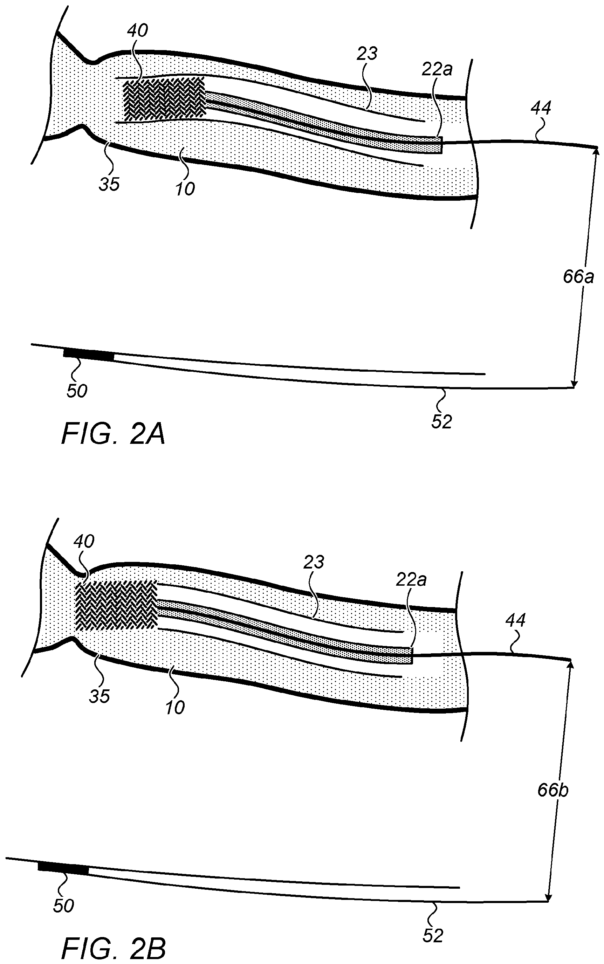

Figs. 2A and 2B are schematic side views of stent 40 positioned inside and outside of a distal end of sheath 23, respectively, in accordance with an embodiment of the present invention. Fig. 2A shows sheath 23 inside a lumen, such as a blood vessel 35, and a distal end 22a of shaft 22 inside sheath 23. As seen, a stent 40 is attached at a distal edge of shaft 22, where stent 40 is in a collapsed configuration inside sheath 23. An insulated electrical wire 44 is electrically connected at its distal edge to stent 40, while patch electrode 50, with a wire 52, is attached to the skin of the patient. Wires 44 and 52 are connected to first pole 63 and second pole 64, respectively, of readout circuitry 65 (as seen in Fig. 1) that measures an impedance 66a between the collapsed stent and electrode patch 50, for example, by flowing electrical current and measuring a voltage falling between the two poles.

Fig. 2B shows distal end 22a after being slightly advanced distally as compared to its shown position in Fig. 2A. As a result, stent 40 is just outside sheath 23 and is expanded. In stent 40 position of Fig. 2B, readout circuitry 65 (seen in Fig. 1) measures a lower impedance 66b (i.e., lower than impedance 66a) between (the possibly slightly expanded) stent 40 in contact with blood 10 and patch electrode 50.

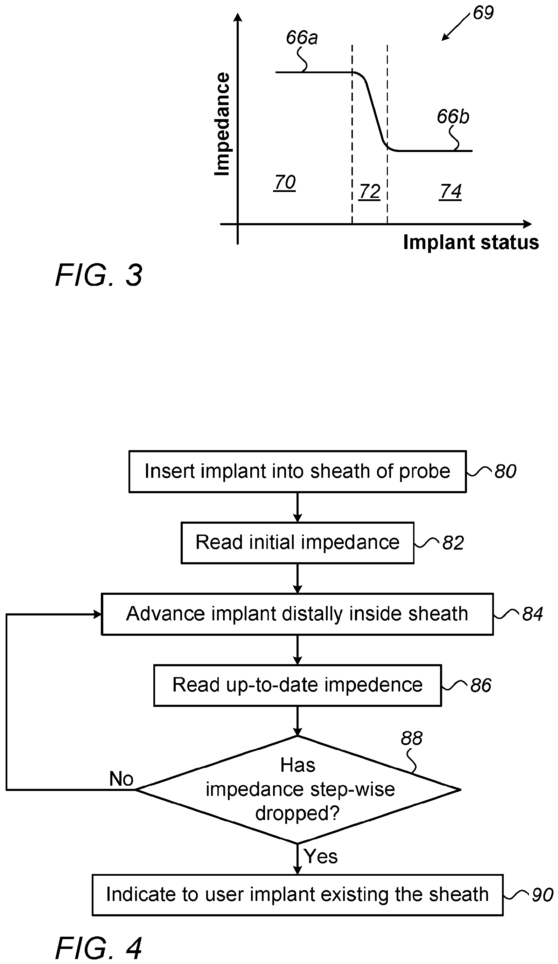

Fig. 3 is a graph plot 69 that schematically shows impedances 66a and 66b measured between the stent of Figs. 2A and 2B and a patch electrode 50, respectively, in accordance with an embodiment of the present invention. Graph plot 69 shows a step-wise drop in impedance between a region 70 that corresponds to stent 40 being collapsed inside sheath 23 and a region 74, in which stent 40 is just outside the sheath, i.e., in contact with blood (and possibly also slightly expanded). The drop in the impedance occurs at a relatively narrow region 72 of implant status, which corresponds to the implant being abruptly exposed to surrounding blood 10 as stent 40 is being advanced and exits sheath 23. Impedances 66a and 66b are usually much larger than the drop, with impedances 66a and 66b measured in hundreds of Ohms each, while the drop is measured in Ohms. The example graph plot shown in Fig. 3 is chosen purely for the sake of conceptual clarity. In practice, an electrical measurement may show a distribution of values, and impedance data points may be fitted with a step-wise curve to assist the indication of the exit of stent 40.

Fig. 4 is a flow chart that schematically describes a method to determine the status of the stent shown in Figs. 2A and 2B, in accordance with an embodiment of the present invention. The process begins with physician 30 inserting the implant (e.g., stent 40) into sheath 23 of catheter 21, at an implant insertion step 80. Readout circuitry 65 reads and records the impedance when stent 40 is still well inside sheath 23 (i.e., in region 70 of impedance readings), at an initial impedance reading step 82.

Physician 30 advances the implant distally through sheath 23, at implant advancement step 84. Readout circuitry 65 reads and records the up-to-date impedance accordingly, at an up-to-date impedance reading step 86.

Either physician 30, or a processor, regularly checks if the impedance has dropped in a step-wise fashion, at an impedance monitoring step 88. If the impedance hasn't yet dropped, the process continues by physician 30 advancing the implant further distally (i.e., process returns to step 86). If there is an indication of a step-wise drop in impedance, the readout circuitry 65, or the processor, indicates to the user that the implant is exiting the sheath, at an implant status indication step 90. In an embodiment, readout circuitry 65 indicates the implant exiting by an audiovisual alert that impedance has made a step-wise drop by at least a prespecified value. The processor may show a visual indication on display 27.

Although the embodiments described herein mainly address cardiac applications, the methods and systems described herein can also be used in other applications, such as in invasive gastroenterology and neurology procedures.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. An apparatus, comprising:
an electrical conductor, which is configured to be connected to an implant fitted at a distal end of a shaft for insertion via a sheath into a liquid-filled lumen of an organ of a patient;
a patch electrode that is configured to be attached to skin of the patient; and
readout circuitry, which is configured to be connected to the electrical conductor and to the patch electrode, to measure, via the electrical conductor, an electrical impedance between the implant and the patch electrode, and to detect the implant exiting the sheath into the lumen by detecting a drop in the electrical impedance that is larger than a predefined threshold.

2. The apparatus according to claim 1, and comprising a magnetic sensor fitted at the distal end of the shaft, adjacently to the implant, the magnetic sensor configured to transmit position signals indicative of a position of the implant when the implant exits the sheath into the lumen.

3. The apparatus according to claim 1, wherein the liquid is blood.

4. The apparatus according to claim 1, wherein the organ is a heart.

5. The apparatus according to claim 1, wherein the implant is an artificial heart-valve.

6. The apparatus according to claim 1, wherein the implant is a stent.

7. A method, comprising:
connecting an electrical conductor to an implant fitted at a distal end of a shaft for insertion via a sheath into a liquid-filled lumen of an organ of a patient;
attaching a patch electrode to skin of the patient; and
measuring, via the electrical conductor, an electrical impedance between the implant and the patch electrode, and detecting the implant exiting the sheath into the lumen by detecting a drop in the electrical impedance that is larger than a predefined threshold.

8. The method according to claim 7, and comprising measuring a position of the implant, when the implant exits the sheath into the lumen, using a magnetic sensor fitted at the distal end adjacently to the implant.

9. The method according to claim 7, wherein the liquid is blood.

10. The method according to claim 7, wherein the organ is a heart.

11. The method according to claim 7, wherein the implant is an artificial heart-valve.

12. The method according to claim 7, wherein the implant is a stent.
